(19) Europäisches Patentamt
European Patent Office
Office européen des brevets

(11) Publication number: **0 096 525**
**B1**

(12) **EUROPEAN PATENT SPECIFICATION**

(45) Date of publication of patent specification: **21.01.87**

(51) Int. Cl.[4]: **A 01 N 59/08,** A 01 N 37/16, C 11 D 3/39, C 11 D 3/48

(21) Application number: **83303099.2**

(22) Date of filing: **31.05.83**

(54) **Sanitizer compositions.**

(30) Priority: **03.06.82 GB 8216152**

(43) Date of publication of application:
**21.12.83 Bulletin 83/51**

(45) Publication of the grant of the patent:
**21.01.87 Bulletin 87/04**

(84) Designated Contracting States:
**AT BE CH DE FR GB IT LI NL SE**

(56) References cited:
**EP-A-0 024 367**
**EP-A-0 027 693**
**FR-A-1 568 918**
**FR-A-2 148 302**

(73) Proprietor: **Interox Chemicals Limited**
**Hanover House 14 Hanover Square**
**London W1R 0BE (GB)**

(72) Inventor: **Smith, Eileen**
**19 Wells Close**
**Mickle Trafford Chester Cheshire (GB)**
Inventor: **Slater, David**
**7 Chapel Road**
**Penketh Warrington Cheshire (GB)**

(74) Representative: **Pearce, Timothy et al**
**Patent Department Laporte Industries Limited**
**P.O. Box 2 Moorfield Road**
**Widnes Cheshire WA8 0HE (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to sanitizer compositions, and in particular to such compositions containing a peroxyacid species, and additionally to processes for sanitization employing such compositions.

The sanitizer compositions described herein are primarily directed towards the sanitization and cleansing of reusable nappies, sometimes referred to as babies napkins and in America as diapers, but such compositions can also be used for other apparel, soiled linen or fabrics or for surfaces which may have come into contact with human or animal excrements. The problems of sanitization of nappies is of especial importance in view of the subsequent intimate and prolonged contact between a reused nappy and the baby and the vulnerability particularly of young babies to infection. Consequently, there is constantly a need to provide new and effective nappy sanitizer compositions. Furthermore, the natural pride of parents in their offspring lead them to seek sanitizing compositions offering a cleansed and bright appearance for the nappies in addition to sanitization.

French Patent Specification No. 1 568 918 to Vick International Limited describes washing and disinfecting compositions for babies nappies which comprise potassium monopersulphate, sodium chloride, a surfactant and detergent builders. Such compositions were available commercially from Richardson Vick in 1982 but when tested performed poorly in a recent test for nappy sanitizing compositions, Australian Standard 2351.

European Patent Specification 27693 to Interox Chemicals Limited describes the preparation of various magnesium salts of percarboxylic acid/carboxylate compounds including magnesium monoperoxyphthalate and washing compositions containing them but makes no reference to the inclusion of an alkali metal chloride in such compositions and indeed on page 6 excludes salts of halogen acids from the class of diluents to employ with the invention magnesium salts.

According to the present invention there is provided a sanitizer composition comprising a peroxygen compound, an alkali metal chloride, surfactant and detergent builder characterised in that it contains from 5 to 20% of magnesium monoperoxyphthalate, 30 to 60% alkali metal chloride, 3 to 15% anionic surfactant, 0 to 20% nonionic ethoxylated surfactant, 10 to 50% detergent builder or builders and 0 to 5% of a complexing builder.

Herein, % is by weight, based on the composition unless stated otherwise.

The components of such compositions are usually included in particulate form, the particle ranges in practice being selected so as to avoid if possible substantial segregation of the components during transport or storage.

It will be observed that invention compositions contain both the peroxyacid compound and an alkali metal chloride, which can interact in aqueous solution to generate oxychlorine species, of which the nature of the predominating species will vary depending upon the pH of the solution.

The alkali metal chloride is preferably sodium chloride in view of its widespread availability in crystalline form. In many invention compositions, the ratio of alkali metal chloride to peroxygen compound is from 1:1 to 20:1.

By magnesium monoperoxyphthalate, we mean herein the compound having the empirical formula

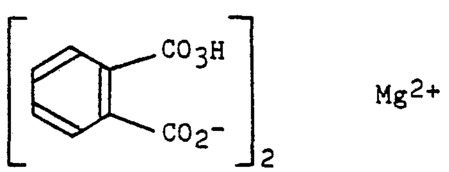

It will be recognised that the salt is that of the carboxylate group only, that the peroxy group remains in acid form. The aforementioned magnesium salt, which for the sake of brevity may be referred to herein alternatively as MMPP, demonstrates good storage stability itself.

The magnesium monoperoxyphthalate can be prepared by the method described in European Patent Specification 27693, the resultant crystalline material being hydrated, x-ray diffraction data indicating a hexahydro magnesium structure.

In practice, the method of manufacture of MMPP often results in the particles of the peroxygen compound containing a small proportion of the corresponding non-peroxygenerated compound. Thus for MMPP, a small proportion of magnesium phthalate can be present.

In order to enhance the cleansing performance of the composition it includes one or more surfactants. The surfactants can be soaps or be synthetic, for example as described in chapter 2 of Synthetic Detergents by A Davidsohn and B M Milwidsky, 5th Edition published in 1972 by Leonard Hill, London, and methods of making them are described in chapter 4 of the same book.

Amongst anionic surfactants described on pages 15—23 of the aforementioned book, sulphonates and sulphates are of special practical importance. The sulphonates includes, for example, alkaryl sulphonates, and particularly alkyl benzene sulphonates, the alkyl group preferably being straight chain containing 9 to 15 carbon atoms, of which one of the most commonly employed surfactants is linear dodecyl benzene

sulphonate. Other anionic sulphonates which are useful in washing compositions containing MMPP include olefin sulphonates, obtained, for example, by sulphonating primary or secondary aliphatic monoolefins, alkene sulphonates, especially linear alkene sulphonates, and hydroxy alkene sulphonates and disulphonates, especially 3-, 4-, and 5-, hydroxy-n-alkyl sulphonates in which the alkyl group contains any even number from 10 to 24 carbon atoms. Other desirable anionic surfactants include alcohol sulphates, preferably linear, having a chain length of at least 10 carbon atoms and sulphated fatty acid alkanolamides. Other sulphates comprise sulphated nonionic surfactants as for example alkylphenyl-ethylene oxide ether sulphate in which the alkyl groups contain from about 8 to 12 carbon atoms and there are 1 to 10 units of ethylene oxide in each molecule. Yet other sulphate surfactants comprise alkyl ether sulphates where the alkyl group contains from 10 to 20 carbon atoms, preferably linearly and each molecule contains from 1 to 10 preferably from 1 to 4 molecules or ethylene oxide. Further anionic surfactants include phosphate derivatives of the ethylene oxide based nonionic surfactants described herein.

Many of the suitable nonionic surfactants comprise condensation products of ethylene oxide and possibly propylene oxide. One class of such nonionic surfactants which is of special importance comprises water soluble condensation products of alcohols containing from 8 to 18 carbon atoms with an ethylene oxide polymer often containing at least 10 molecules of ethylene oxide per molecule of surfactant, e.g. from 10 to 30 moles of ethylene oxide. Particularly desirable nonionic surfactants comprise water soluble condensates of alkyl phenols or alkyl naphthols with an ethylene oxide polymer normally containing from 5 to 25 moles of ethylene oxide per mole of alkyl phenol or alkyl naphthol. The alkyl group normally contains from 6 to 12 carbon atoms and is frequently linear.

As an alternative to the hydrophobic moiety of the nonionic surfactant being linked to the hydrophilic moiety by an ether link as in alkyl phenol ethylene oxide condensates, the linkage can be an ester group. The hydrophobic moiety is normally the residue of a straight chain aliphatic acid containing from 10 to 22 carbon atoms and more particularly lauric, stearic and oleic residues. In one class of nonionic ester surfactants, the hydrophilic moiety comprises polyethylene oxide, frequently in the ratio of from 5 to 30 moles of ethylene oxide per mole of the fatty acid residue. It will be recognised that both mono and di esters can be employed. Alternatively it is possible to employ as the hydrophilic moiety glycerol, thereby producing either mono- or di-glycerides. In a further group, the hydrophilic moiety comprises sorbitol. A further class of nonionic surfactants comprise alkanolamides in which a C10 to C22 amide is condensed with a polyethylene oxide or polypropylene glycol hydrophilic moiety or moieties. Semi-polar detergents include water soluble amine oxides, water soluble phosphine oxides and water soluble sulphur oxides, each containing one alkyl moiety of from 10 to 22 carbon atoms and two short chain moieties selected from the groups of alkyl and hydroxyalkyl groups containing 1 to 3 carbon atoms.

The nonionic and anionic surfactants are often employed together, in many cases in a weight ratio within the range 2:1 to 1:10.

The surfactants provide in total in many embodiments from 1 to 25% of the composition.

A further component of the compositions of the present invention is a builder or mixture of builders, elsewhere alternatively referred to as detergent builders. It will be understood that such compounds can perform several different roles during use of the composition, including one or more of detergent enhancement, peptisation, water softening, sequestration of catalytic metals, pH adjustment, and percompound stabilisation. The skilled chemist in cleansing, bleaching and sanitizing compositions will naturally recognise which of the foregoing roles are provided by each of the subsequently listed builders, and he will of course select the builder and the form of the builder, i.e. either acid or alkaline salt form in accordance with standard knowledge to obtain his desired pH in the sanitizing solution and his desired combination of properties. Many of the suitable builders are water soluble. These include alkali metal phosphates, often being pyrophosphates, tripolyphosphates, or higher polymetaphosphates, the latter of which include hexametaphosphates and alkali metal hydrogen phosphates. They also include alkali metal carbonates and bicarbonates and borates. Water insoluble inorganic builders include zeolites, especially zeolites A and X. As is conventional, the alkali metal is preferably sodium in such builders.

The builder can also be organic and such builders include hydroxycarboxylic acids, polycarboxylic acids, aminocarboxylic acids and phosphonic acids or their alkali metal, especially sodium salts thereof. Such organic builders are often loosely referred to as complexing builders. It will be recognised that the classes overlap to some extent. Suitable examples of such complexing builders, listed in acid form for brevity, include $C_2$ to $C_{10}$ alpha-omega dicarboxylic acids, 1,1,3,3-propane tetracarboxylic acid, oxydiacetic acid, citric acid, tartaric acid, gluconic acid, oxydisuccinic acid, furan tetracarboxylic acid and polymeric carboxylic acid compounds obtained from e.g. acrylic acid, hydroxyacrylic acid or maleic acid. Other examples include nitrilotriacetic acid (NTA), ethylenediaminetetraacetic acid (EDTA), diethylenetriaminepentaacetic acid (DTPA) and their counterparts in which the acetic acid group is replaced by a 2-hydroxyethyl group, or a methylene (phosphonic acid) group (NTMP, EDTMP, DTPMP). Further examples include 1-hydroxyethane-1,1-diphosphonic acid (HEPA). These exemplified builders are representative of the various sub-classes of builders, and often they are used as the sodium salt.

The total amount of builder is in the range of 10 to 50%, of which at least a part is a phosphate in many embodiments in view of its combination of useful properties, and often from 5 to 25% of the composition. The complexing builders, when used, tend to be selected within the range of 0.1% to 5% of the composition, and often are included as a complement to the inorganic builder or builders.

In some preferred embodiments, the composition comprises:

(a) from 5 to 20% of MMPP;

(b) from 30 to 60% sodium chloride;

(c1) from 3 to 15% anionic surfactant, such as a linear $C_9$—$C_{15}$ alkyl benzene sulphonate, sodium salt;

(c2) from 0 to 20% of a nonionic ethoxylated surfactant; and

(d1) from 5 to 30% of a phosphate builder, sodium salt;

(d2) from 5 to 30% of a non-phosphate inorganic builder, such as sodium carbonate or bicarbonate or borax; and

(d3) from 0 to 5% of an organic complexing builder such as NTA, EDTA, EDTMP, DTPMP or gluconate or mixture of two or more thereof.

In addition to the foregoing components, the composition can include also one or more auxiliary agents, such as alkaline sulphates — e.g. sodium sulphates from 0 to 40% of the composition and in minor amounts soil anti-redeposition agents such as CMC or PVP, dyes, perfumes, dye transfer inhibitors and optical brightening agents. These minor auxiliary components often comprise from 0 to 20% in total, in many cases from 1 to 10%. In this context it will be understood that small concentrations of phosphonates, gluconate and aminocarboxylates alone or in combination in a total amount of for example as low a range as 0.2 to 2.0% can stabilise the peroxygen compound in solution. Other peroxygen stabilisers like dipicolinic acid or hydroxy quinoline can also be used.

The compositions are under normal storage conditions in the form of mixtures of solid particles. Accordingly they can be made in conventional equipment for blending and mixing powders. It will also be recognised that the components other than the peroxygen compound can be pre-blended together, to any desired extent, in slurry which is subsequently dried, e.g. spray dried and thereafter blended with the peroxygen compound. Accordingly also, the surfactant can often be introduced into the pre-mixture in liquid form.

The compositions are employed in a dilute aqueous solution, often at a concentration of 1 to 20 g/l, in which many of them generate a pH preferably within the range of pH 7.8 to 11. The solution is often made at up to hand hot temperatures, such as from 25 to 45°C, although higher temperatures could be used and thereafter allowed to cool to ambient or maintained within that range. The solution can be replaced periodically, such as daily and if desired the concentration of sanitizer composition can be augmented from time to time, e.g. to restore the available oxygen level to around its original level. For more general washing uses any temperature from ambient to boiling point can be employed, preferably using a solution having an initial avox of at least 5 ppm. For cleaning many surfaces a slurry or paste can be used instead of a solution, if desired.

Prolonged contact between the aqueous mixtures of the invention compositions and steel surfaces is not recommended.

Having described the invention in general terms, an embodiment thereof will now be given in greater detail by way of example only.

## Examples

In the Examples, sanitizer compositions were produced by blending together in a mixer, dry powders for a, b, d1 and d2 and an aqueous solution of c to give on a dry weight basis the following compositions:

(a) magnesium monoperoxyphthalate (avox of 5.9% w/w of the MMPP granules)

(b) sodium chloride

(c) linear dodecyl benzene sulphonate (NANSA SS30)

(d1) sodium tripolyphosphate

(d2) sodium carbonate

(d3) sodium bicarbonate

TABLE 1

| | % weight/weight in Example No | | | | | |
|---|---|---|---|---|---|---|
| Component | 1 | 2 | 3 | 4 | 5 | 6 |
| (a) | 10 | 12 | 7 | 10 | 17 | 15 |
| (b) | 50 | 48 | 53 | 50 | 53 | 55 |
| (c) | 7 | 7 | 7 | 7 | 7 | 7 |
| (d1) | 10 | 10 | 10 | 10 | 10 | 10 |
| (d2) | 23 | 23 | 23 | 3 | 2 | 1 |
| (d3) | — | — | — | 20 | 21 | 22 |

In Examples 1 to 3, the solution obtained had a pH of over 10, and in Examples 4 to 6 of about pH 8.1.

The sanitizing capability of each composition was tested by dissolving it at 37°C initial temperature in water at 4.4 g/l, and determining the survival of standard bacteria, Escherichia Coli (NCTC 8196) and Staphylcoccus Aureus (NCTC 4163) under the stimulated use conditions of the Standards Association of Australia, Australian Standard 2351 — 1980 Nappy Sanitizers. The results of faecal challenges at the Start ($t_0$) and End ($t_5$), measured at time 7 hours are given below. Certain minor modifications were made to the standard procedure, namely the use of innoculated agar slopes instead of a centrifuged broth to provide the bacterial suspension, and the use of the pour plate technique using 1 ml aliquots introduced, incubated at 37°C for 48 hours, instead of the spread dry technique, 0.5 ml aliquots and incubation at 30°C for 72 hours.

In the tests, a 700 ml sample of sanitizing solution was used with 6 nappies.

By way of comparison in comparison A, a commercial nappy sanitizer composition of similar composition, but containing an inorganic peracid salt instead of MMPP, was tested using the same modified procedure and conditions. The commercial product was analysed to approximately:

| | |
|---|---|
| Persulphate triple salt | 11.5% |
| Sodium chloride | 53% |
| Sodium tripolyphosphate | 11% |
| Sodium carbonate | 10% |
| Borates | 3% |
| Surfactants and other organic components | 8% |

The persulphate triple salt has the formula $2KH.SO_5.K_2SO_4.KHSO_4$ and has a nominal 6% avox.

TABLE 2

| Product Used | Faecal Challenge | Bacterial Concn at 7 hours E. Coli | S. Aureus |
|---|---|---|---|
| Invention Example 1 | Start | −10 | −10 |
| | End | $1.9\times10^4$ | 80 |
| Comparison A | Start | −10 | −10 |
| | End | $2.4\times10^6$ | $1.6\times10^6$ |
| Control | | $2.9\times10^7$ | $8.7\times10^6$ |

The initial bacterial concentrations were always about 2.5 to $3.4\times10^7$ CFU/cm$^3$, and −10 indicates that the concentration was below 10.

From Table 2, it can be seen that the invention product performed substantially better than the commercial peracid-containing comparison product A. Similar results were obtained on repetition of the trials.

Examples 2 to 6 were compared using the same test procedure with a further commercially available sanitizing composition (B) which on analysis was very similar to the formulation on page 9 of European Patent Specification 47015 (Richardson-Vicks Pty) containing over 25% w/w sodium percarbonate, i.e. an avox content of approximatley 6 times that of Example 1.

The results are summarised in Table 3

TABLE 3

| Product Used | Avox % | Faecal Challenge | Bacterial Concn — 7 hours E. Coli | S. Aureus |
|---|---|---|---|---|
| Ex. 2 | 0.72 | end | $6.7\times10^3$ | $1.2\times10^4$ |
| Control | | | $5.0\times10^7$ | $6.7\times10^6$ |
| Ex. 3 | 0.42 | end | $5.7\times10^3$ | $8.8\times10^2$ |
| Control | | | $1.3\times10^7$ | $1.3\times10^7$ |
| Ex. 4 | 0.6 | end | 10 | 10 |
| Control | | | $2.2\times10^7$ | $1.0\times10^7$ |
| Ex. 5 | 0.42 | end | $9.7\times10^3$ | $1\times10^4$ |
| Control | | | $3.3\times10^7$ | $7.5\times10^6$ |
| Ex. 6 | 0.3 | end | $2.2\times10^4$ | $3.6\times10^3$ |
| Control | | | $1.1\times10^7$ | $6.8\times10^6$ |
| Comparison B | 3.8 | end | $2.6\times10^4$ | $2.9\times10^5$ |
| Control | | | $2.2\times10^7$ | $5.9\times10^6$ |
| Comparison B | 3.8 | end | $1.2\times10^5$ | $1.5\times10^4$ |
| Control | | | $2.3\times10^7$ | $7.2\times10^6$ |

From Table 2, it can be seen that the invention products within the limits imposed by microbiological test procedures were demonstrably better than comparison B even though the latter contained often about 10 times as much avox (active oxygen). It was also noticed that for many of the Example compositions, if a slightly longer soak (an extra 1 or 2 hours) was used, a further substantial fall in bacterial count to below or near 100 CFU $cm^{-3}$ occurred.

**Claims**

1. A sanitizer composition comprising a peroxygen compound, an alkali metal chloride, surfactant and detergent builder characterised in that it contains from 5 to 20% of magnesium monoperoxyphthalate, 30 to 60% alkali metal chloride, 3 to 15% anionic surfactant, 0 to 20% nonionic ethoxylated surfactant, 10 to 50% detergent builder or builders and 0 to 5% of a complexing builder.

2. A composition according to claim 1 characterised by containing 5 to 30% of a non-phosphate builder and 5 to 30% of a phosphate builder.

3. A composition according to claim 2 characterised in that the non-phosphate builder is sodium carbonate and/or bicarbonate.

4. Use of a composition according to any preceding claim in solution for sanitising an article especially a nappy, brought into contact therewith.

**Patentansprüche**

1. Eine Desinfektionsmittel-Zusammensetzung enthaltend eine Persauerstoff-Verbindung, ein Alkalimetallchlorid, oberflächenaktive Substanzen und Builder für Detergentien, dadurch gekennzeichnet, daß sie 5 bis 20% Magnesiummonoperoxyphthalat, 30 bis 60% Alkalimetallchlorid, 3 bis 15% anionische oberflächenaktive Substanz, 0 bis 20% nicht-ionische äthoxylierte oberflächenaktive Substanz, 10 bis 50% eines oder mehrerer Builder für Detergentien und 0 bis 5% eines komplexbildenden Builders.

2. Eine Zusammensetzung gemäß Anspruch 2, dadurch gekennzeichnet, daß sie 5 bis 30% eines phosphatfreien Builders und 5 bis 30% eines Phosphat-Builders enthält.

3. Eine Zusammensetzung nach Anspruch 2, dadurch gekennzeichnet, daß der phosphatfreie Builder Natriumcarbonat und/oder -bicarbonat ist.

4. Verwendung einer Zusammensetzung gemäß einem der vorstehenden Ansprüche in Lösung für die Desinfektion eines Gegenstandes, insbesondere einer Babywindel, welcher mit der Zusammensetzung kontaktiert worden ist.

**Revendications**

1. Composition désinfectante comprenant un composé peroxygéné, un chlorure de métal alcalin, un agent tensioactif et un adjuvant de détergence (builder) caractérisée en ce qu'elle contient de 5 à 20% de

monoperoxyphtalate de magnésium, 30 à 60% de chlorure de métal alcalin, 3 à 15% d'un agent tensio-actif anionique, 0 à 20% d'un agent tensio-actif non ionique éthoxylé, 10 à 50% d'un ou plusieurs adjuvant(s) de détergence (builder) et 0 à 5% d'un adjuvant (builder) complexant.

2. Composition suivant la revendication 1 caractérisée en ce qu'elle contient de 5 à 30% d'un adjuvant de détergence (builder) non phosphaté et 5 à 30% d'un adjuvant de détergence phosphate.

3. Composition suivant la revendication 2 caractérisée en ce que l'adjuvant de détergence non phosphaté est du carbonate et/ou du bicarbonate de sodium.

4. Emploi d'une composition, suivant l'une quelconque des revendications précédentes, en solution pour la désinfection d'un article, particulièrement un lange, mis en contact avec elle.